# EUROPÄISCHE PATENTSCHRIFT

(11) **EP 2 651 383 B1**
(45) Veröffentlichungstag und Bekanntmachung des Hinweises auf die Patenterteilung: **31.05.2017**
(21) Anmeldenummer: 11801651.8
(22) Anmeldetag: 05.12.2011
(51) Int. Cl.: A61K 8/34, A61Q 5/06, A61K 8/92

(54) **TEMPORÄRE UMFORMUNG KERATINHALTIGER FASERN DURCH VERWENDUNG MINDESTENS EINES SPEZIELLEN CYCLOALKAN-DERIVATS**
TEMPORARY SHAPING OF KERATIN-CONTAINING FIBRES USING AT LEAST ONE SPECIAL CYCLOALKANE DERIVATIVE
MISE EN FORME TEMPORAIRE DE FIBRES KÉRATINIQUES PAR UTILISATION D'AU MOINS UN DÉRIVÉ SPÉCIFIQUE D'UN CYCLOALCANE

(30) Priorität: 16.12.2010 DE 102010063247
(43) Veröffentlichungstag der Anmeldung: 23.10.2013
(73) Patentinhaber: Henkel AG & Co. KGaA, 40589 Düsseldorf (DE)
(72) Erfinder: BANOWSKI, Bernhard, 40597 Düsseldorf (DE); KNAPPE, Thorsten, 22869 Schenefeld (DE)
(86) Internationale Anmeldenummer: PCT/EP2011/071726
(87) Internationale Veröffentlichungsnummer: WO 2012/080016

(56) Entgegenhaltungen:
- EP-A1- 1 588 689
- EP-A2- 1 287 812
- US-A- 4 873 079
- US-A1- 2010 158 821
- US-B1- 6 749 836

## Beschreibung

Die vorliegende Erfindung betrifft Hilfsmittel für die temporäre Umformung keratinhaltiger Fasern. Gegenstand der Erfindung ist daher die Verwendung eines speziellen Cycloalkans zur temporären Umformung keratinhaltiger Fasern, insbesondere menschlicher Haare. Zudem sind bevorzugt geeignete Mittel für die temporäre Umformung keratinhaltiger Fasern Gegenstand der Erfindung. Diese Mittel enthalten eine Kombination aus mindestens einem speziellen Cycloalkan und mindestens einem festigenden Wirkstoff. Außerdem ist die Verwendung besagter Mittel und ein entsprechendes Umformungsverfahren durch Anwendung der besagten speziellen Cycloalkane Gegenstand der Erfindung.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Während bei der permanenten Umformung die chemische Struktur der keratinhaltigen Faser durch Reduktion und Oxidation modifiziert wird, findet bei der temporären Umformung keine solche Modifikation des chemischen Struktur statt. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung keratinischer Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde bereits als festigende Wirkstoffe eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden.

Oftmals wirkt das mit einem fixierenden Wirkstoff behandelte Haar stumpf und wenig glänzend. Werden wie üblich zusätzlich Öle als Glanzgeber eingesetzt, wirkt der erzielte Haarglanz oft unnatürlich fettig. Der Halt wird durch die zusätzlichen glanzgebenden Öle meist herabgesetzt. Ferner lassen sich Öle nur mit Shampoo aus dem Haar waschen.

Stylingmittel zu entwickeln, die alle gewünschten Eigenschaften in Kombination aufweisen, bereitet nach wie vor Schwierigkeiten. Insbesondere gilt dies für die Kombination von starkem Halt einerseits, und Auswaschbarkeit sowie Remodellierbarkeit andererseits.

Aufgabe der vorliegenden Erfindung war es daher, ein Mittel zur temporären Verformung keratinhaltiger Fasern zur Verfügung zu stellen, das sich durch einen hohen Haltegrad bzw. durch eine hohe Pflegewirkung auszeichnet, sich gut wieder von der Faser abziehen (beispielsweise durch Spülen) lässt.

Es wurde nunmehr gefunden, dass kosmetische Mittel, enthaltend das spezielle Cycloalkanderivat, eine haltbare und flexible Formgebung keratinhaltiger Fasern ermöglichen. Die durch das Mittel den Fasern aufgeprägte Form lässt sich, insbesondere unter Einwirkung von Wärme, ohne erneute Applikation eines kosmetischen Mittels, durch Umformung zu einer neuen Form modellieren. Die mit Hilfe des besagten Mittels umgeformten keratinhaltigen Fasern wirken nicht matt oder stumpf, sondern besitzen einen natürlichen Glanz. Das besagte Mittel lässt sich gut auswaschen. Die erfindungsgemäßen Effekte werden insbesondere durch eine spezielle Ausführungsform der Erfindung in Gestalt des Mittels verbessert.

Das Cycloalkan 1,4-Bis(hydroxymethyl)cyclohexan ist Komponente (a) des zu verwendenden Mittels. Dabei eignen sich *cis*-1,4-Bis(hydroxymethyl)cyclohexan (Schmelzpunkt: 43 °C) und *trans*-1,4-Bis(hydroxymethyl)cyclohexan (Schmelzpunkt: 67°C) oder eine Mischung der cis/trans-Isomere.

Es hat sich als erfindungsgemäß bevorzugt herausgestellt, wenn die in dem erfindungsgemäßen Mittel enthaltenen Cycloalkan einen Schmelzpunkt von 35°C bis 95°C, insbesondere 40°C bis 85 °C, besitzt Schmelzpunkte werden im Rahmen der Erfindung bei 1013 mbar gemessen.

Die Mittel enthalten das Cycloalkan bevorzugt in einer Menge von 0,5 bis 20,0 Gew.-%, insbesondere von 1,0 bis 10,0 Gew.-%, jeweils bezogen auf das Gewicht des Mittels, enthalten.

Die Mittel enthalten die Inhalts- bzw. Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, berechnet auf das Gesamtgewicht des Mittels.

Besonders bevorzugt enthält der kosmetische Träger Wasser (insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser) enthält.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Es ist erfindungsgemäß bevorzugt mindestens ein (C₁ bis C₄)-Monohydroxyalkan in den Mitteln insbesondere in einer Menge von 1 bis 50 Gew.-% insbesondere von 5 bis 30 Gew.-% einzusetzen. Dies ist wiederum insbesondere für die Konfektionierung als Pumpschaum oder Aerosolschaum bevorzugt.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Es sind solche kosmetischen Mittel besonders bevorzugt, in denen die Cycloalkane in dem kosmetischen akzeptablen Träger vollständig gelöst, vorliegen. Die bevorzugten kosmetisch akzeptablen Träger (*vide supra*) gelten im Rahmen dieser Ausführungsform ebenso als bevorzugt.

Die Mittel enthalten zwingend als zweite Komponente mindestens einen festigenden Wirkstoff. Dieser festigende Wirkstoff ist von den Cycloalkanderivaten verschieden. Ein festigender Wirkstoff im Sinne der Erfindung trägt im Rahmen der temporären Umformung keratinhaltiger Fasern zum Halt der aufgeprägten Form der Fasern (bei Haaren insbesondere der Halt einer Frisur oder des Haarvolumens) bei. Als eine Testmethode für die festigende Wirkung eines Wirkstoffs wird häufig der so genannte curl-retention - Test angewendet.

Bevorzugte Mittel enthalten die filmbildenden Wirkstoffe in einer Menge von 0,1 Gew.-% bis 25,0 Gew.-%, besonders bevorzugt von 1,0 Gew.-% bis 20,0 Gew.-%, ganz besonders bevorzugt von 1,0 Gew.-% bis 10,0 Gew.-%, jeweils bezogen auf das Gewicht des Mittels. Der festigende Wirkstoff des Mittels wird bevorzugt ausgewählt aus mindestens einem festigenden Polymer und/oder aus mindestens einem Wachs.

Im Rahmen einer allgemeinen Ausführungsform der Erfindung ist es bevorzugt, dass der festigende Wirkstoff ausgewählt wird aus mindestens einem festigenden Polymer aus der Gruppe, die gebildet wird aus festigenden nichtionischen Polymeren, festigenden anionischen Polymeren, festigenden amphoteren Polymeren und festigenden kationischen Polymeren.
Zusätzlich kann das erfindungsgemäße Mittel bevorzugt mindestens ein festigendes kationisches Polymer enthalten.

Die zusätzlichen festigenden kationischen Polymere weisen mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.

Im Sinne dieser Ausführungsform bevorzugte Mittel enthalten die filmbildenden kationischen und/oder festigenden kationischen Polymere in einer Menge von 0,1 Gew.-% bis 20,0 Gew.-%, besonders bevorzugt von 0,2 Gew.-% bis 10,0 Gew.-%, ganz besonders bevorzugt von 0,5 Gew.-% bis 5,0 Gew.-%, jeweils bezogen auf das Gewicht des Mittels.

Die kationischen festigenden Polymere können erfindungsgemäß aus kationischen, quaternisierten Cellulose-Derivaten ausgewählt werden.

Es erweisen sich generell solche kationischen, quaternisierten Cellulosen als im Sinne der Ausführungsform vorteilhaft, die in einer Seitenkette mehr als eine permanente kationische Ladung tragen.

Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Weiterhin eignen sich solche kationischen festigenden Polymere, die mindestens eine Struktureinheit der Formel (I) und mindestens eine Struktureinheit der Formel (VI) und gegebenenfalls mindestens eine Struktureinheit der Formel (V) umfassen 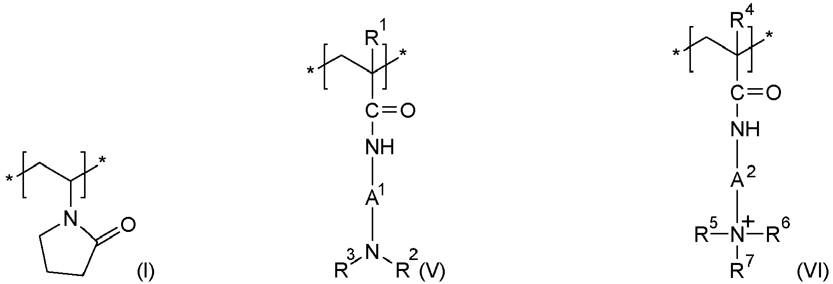 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Geeignete Verbindungen sind beispielsweise als
- Copolymere aus mit Diethylsulfat quaterniertem Dimethylaminoethylmethacrylat, mit N-Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus Methacryloylaminopropyllauryldimethylammonium chlorid mit N-Vinylpyrrolidon und Dimethylaminopropylmethacrylamid mit der INCI-Bezeichnung Polyquaternium-55 unter den Handelsnamen Styleze^{®} W-10, Styleze^{®} W-20 (Firma ISP),
- Copolymere aus Methacryloylaminopropyllauryldimethylammonium chlorid mit N-Vinylpyrrolidon, N-Vinylcaprolactam und Dimethylaminopropylmethacrylamid mit der INCI-Bezeichnung Polyquaternium-69 unter den Handelsnamen Aquastyle^{®} 300 (Firma ISP),
im Handel erhältlich.

Als im Sinne der Ausführungsform besonders bevorzugt verwendbare filmbildende und/oder festigende Polymere ausgewählt aus kationischen Polymeren die mindestens eine Struktureinheit enthalten, die ein permanent kationisiertes Stickstoffatom aufweisen, dienen weiterhin solche kationischen filmbildenden und/oder kationischen festigenden Copolymere, die mindestens ein Strukturelement der Formel (M1) enthalten 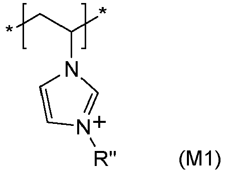 worin
R für eine (C₁ bis C₄)-Alkylgruppe, insbesondere eine Methylgruppe, steht,
und zusätzlich mindestens ein weiteres kationisches und/oder nichtionisches Strukturelement aufweisen.

Es ist wiederum im Rahmen dieser Ausführungsform erfindungsgemäß bevorzugt, wenn in dem erfindungsgemäßen als kationisches filmbildendes und/oder kationisches festigendes Polymer mindestens ein Copolymer (c1) enthalten ist, das neben mindestens einem Strukturelement der Formel (M1) zusätzlich ein Strukturelement der Formel (I) umfasst 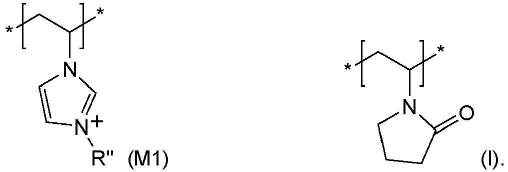 worin
R für eine (C₁ bis C₄)-Alkylgruppe, insbesondere eine Methylgruppe, steht.

Ganz besonders bevorzugte kationische filmbildende und/oder kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.-% Struktureinheiten gemäß Formel (I).

Hierbei ist besonders bevorzugt, wenn die Copolymere (c1) neben Polymereinheiten, die aus dem Einbau der genannten Struktureinheiten gemäß Formel (M1) und (I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M1) mit R" = Methyl und (I) aufgebaut und lassen sich durch die allgemeine Formel (Poly1) 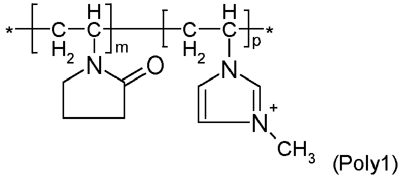 beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (I) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (VII) aufweisen

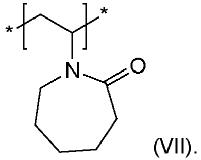

Weitere besonders bevorzugte erfindungsgemäße Mittel dieser Ausführungsform sind somit dadurch gekennzeichnet, daß sie als kationisches filmbildendes und/oder kationisches festigendes Polymer mindestens ein Copolymer (c2) enthalten, das mindestens eine Struktureinheit gemäß Formel (M1-a) und mindestens Struktureinheit gemäß Formel (I) und mindestens Struktureinheit gemäß Formel (VII) enthält

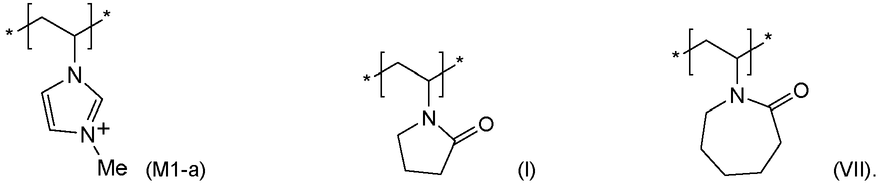

Auch hierbei ist es im Rahmen dieser Ausführungsform besonders bevorzugt, wenn die Copolymere (c2) neben Polymereinheiten, die aus dem Einbau der genannten Struktureinheiten gemäß Formel (M1-a), (I) und (VII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M1-a), (I) und (VII) aufgebaut und lassen sich durch die allgemeine Formel (Poly2) 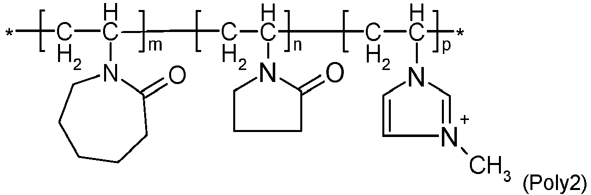 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (I) und 40 bis 60 Mol.-%, vorzugsweise 45 bis 55 Mol.-% und insbesondere 60 Mol.-% Struktureinheiten gemäß Formel (VII).

Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M1-a) und (I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel dieser Ausführungsform sind dadurch gekennzeichnet, daß sie als zusätzliches kationisches filmbildendes und/oder kationisches festigendes Polymer mindestens ein Copolymer (c3) enthalten, das mindestens eine Struktureinheit gemäß Formel (M1-a) und mindestens weitere Struktureinheit gemäß Formel (I) und mindestens weitere Struktureinheit gemäß Formel (VIII) enthält und mindestens weitere Struktureinheit gemäß Formel (IX) enthält

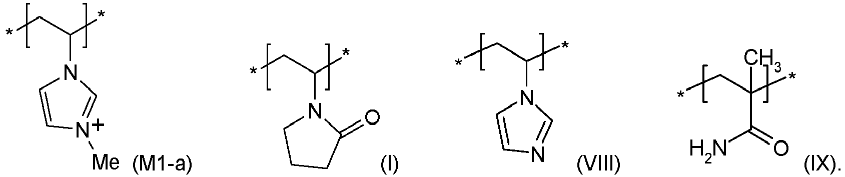

Auch hierbei ist es im Rahmen dieser Ausführungsform besonders bevorzugt, wenn die Copolymere (c3) neben Polymereinheiten, die aus dem Einbau der genannten Struktureinheiten gemäß Formel (M1-a), (I), (VIII) und (IX) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M1-a), (I), (VIII) und (IX) aufgebaut und lassen sich durch die allgemeine Formel (Poly3) 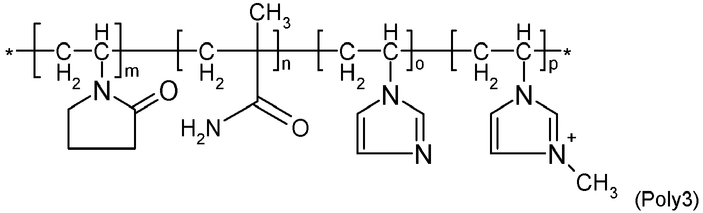 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (I), (VIII) und (IX) im Molekül statistisch verteilt vorliegen.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (I) und 1 bis 20 Mol.-%, vorzugsweise 5 bis 15 Mol.-% und insbesondere 10 Mol.-% Struktureinheiten gemäß Formel (VIII) und 20 bis 40 Mol.-%, vorzugsweise 25 bis 35 Mol.-% und insbesondere 29 Mol.-% Struktureinheiten gemäß Formel (IX).

Unter den zusätzlichen filmbildenden kationischen und/oder festigenden Polymer ausgewählt aus den kationischen Polymeren mit mindesten einem Strukturelement der obigen Formel (M1), gelten als bevorzugt:
- N-Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)),
- N-Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- N-Vinylpyrrolidon/N-Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- N-Vinylpyrrolidon/Methacrylamid/N-Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
sowie Gemische aus diesen Polymeren.

Das Mittel kann als zusätzliches festigendes Polymer mindestens ein festigendes nichtionisches Polymer enthalten. Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die festigenden nichtionischen Polymere sind in dem erfindungsgemäßen Mittel dieser Ausführungsform bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten.

Es sind erfindungsgemäß solche festigende nichtionische Polymere mit mindestens einem Strukturelement der Formel (M2) 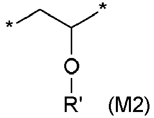 bevorzugt geeignet, welche gemäß Formel (M2) als R' ein Wasserstoffatom, eine Acetylgruppe oder eine Propanoylgruppe, insbesondere eine Acetylgruppe, tragen.

Die festigenden nichtionischen Polymere werden wiederum bevorzugt ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens.

Geeignete Polyvinylpyrrolidone sind beispielsweise Handelsprodukte wie Luviskol^{®} K 90 oder Luviskol^{®} K 85 der Firma BASF SE. Geeignete Polyvinylalkohole werden beispielsweise unter den Handelsbezeichnungen Elvanol^{®} von Du Pont oder Vinol^{®} 523/540 von der Firma Air Products vertrieben.

Geeignetes Polyvinylacetat wird beispielsweise unter dem Handelsnamen Vinac^{®} als Emulsion von der Firma Air Products vertrieben.

Mittel, die als festigendes nichtionisches Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid,
sind erfindungsgemäß ganz besonders bevorzugt.

Weitere mögliche Mittel der Ausführungsform mit zusätzlichem nichtionischen festigendem Polymer sind dadurch gekennzeichnet, daß sie als nichtionisches festigendes Polymer mindestens ein Copolymer enthalten, das mindestens weitere Struktureinheit gemäß Formel (I) und mindestens eine Struktureinheit gemäß Formel (VII) und mindestens eine Struktureinheit gemäß Formel (VIII) enthält

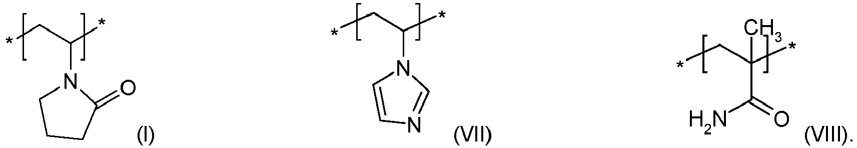

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c4) ausschließlich aus Struktureinheiten der Formel (M1-a), (I), (VII) und (VIII) aufgebaut und lassen sich durch die allgemeine Formel (Poly4) 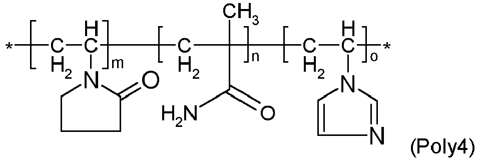 beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Ein besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Die Mittel können als festigendes Polymer auch mindestens ein festigendes amphoteres Polymer enthalten. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO-- oder -SO₃⁻-Gruppen enthalten, und solche Polymere zusammengefasst, die -COOH- oder SO₃H-Gruppen und quartäre Ammoniumgruppen enthalten.

Ein Beispiel für ein einsetzbares festigendes amphoteres Polymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.

Letztere weisen zusätzlich zu der kationogenen Gruppe bzw. positiv geladenen Gruppe mindestens eine negativ geladene Gruppe im Molekül auf und werden auch als zwitterionische Polymere bezeichnet.

Die festigenden amphoteren Polymere sind in den erfindungsgemäßen Mitteln bevorzugt in Mengen von 0,1 Gew.-% bis 20 Gew.-%, besonders bevorzugt von 0,05 bis 10 Gew.-%, bezogen auf das gesamte Mittel, enthalten. Mengen von 0,1 bis 5 Gew.-% sind ganz besonders bevorzugt.

Weiterhin können als festigende Polymere mindestens ein filmbildendes anionisches Polymer eingesetzt werden.

Bei anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte festigende anionische Polymere sind Acrylsäure-Acrylamid-Copolymere sowie insbesondere Polyacrylamidcopolymere mit Sulfonsäuregruppen-haltigen Monomeren. Ein besonders bevorzugtes festigendes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte festigende anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Weitere bevorzugt einsetzbare festigende anionische Polymere werden ausgewählt aus mindestens einem Polymer der Gruppe, die gebildet wird, aus
- Copolymeren aus Vinylacetat und Crotonsäure (wie sie beispielsweise als Handelsprodukt Aristoflex^{®} A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex^{®} Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INCI-Bezeichung Polyurethane-1 von der Firma BASF SE vertrieben wird).

Als festigender Wirkstoff finden bevorzugt Wachse ihren Einsatz. Im Rahmen der Erfindung eingesetzte Wachse sind bei 20°C knetbar, fest bis brüchig hart, grob bis feinkristallin, durchscheinend bis opak, jedoch nicht glasartig; über 40 °C ohne Zersetzung schmelzend. Wachse unterscheiden sich von ähnlichen synthetischen oder natürlichen Produkten (z. B. Harzen, plastischen Massen, Metallseifen usw.) darin, dass sie von 40°C bis 90 °C in den schmelzflüssigen, niedrigviskosen Zustand übergehen.

Es sind erfindungsgemäß solche Wachse bevorzugt, die bei 1013 mbar einen Schmelzpunkt im Bereich von 50 °C bis 85 °C, insbesondere von 60°C bis 75 °C, aufweisen.

Bevorzugt werden die Wachse aus pflanzlichen, tierischen und mineralischen Wachsen ausgewählt. Erfindungsgemäß besonders bevorzugte Wachse sind Bienenwachs (Cera Alba), Carnaubawachs, Candelillawachs, Montanwachs, mikrokristalline Wachse (mikrokristalline Paraffine) und Cetylpalmitat. Die erfindungsgemäße Lehre umfasst auch den kombinierten Einsatz von mehreren Wachsen als Komponente (b) des erfindungsgemäßen Mittels.

Die Zubereitungen enthalten die Wachse vorzugsweise in Mengen von 1,5 bis 60 Gew.-%, bezogen auf die gesamte Zubereitung. Mengen von 5 bis 40 Gew.-%, insbesondere von 10 bis 25 Gew.-%, sind besonders bevorzugt.

Es hat sich als besonders bevorzugt erwiesen, wenn die Mittel zusätzlich mindestens ein nichtionisches Tensid enthalten.
Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoft kann beispielsweise ein Silikonöl und/oder ein Silikongum eingesetzt werden. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Siliciumorganischer Verbindungen. Zunächst werden hierunter die Dimethiconole verstanden.

Dimethicone bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning ^{®} 5330 Fluid vertrieben.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Besonders geeignete Silikone sind aminofunktionelle Silikone, insbesondere die Silikone, die unter der INCI-Bezeichnung Amodimethicone zusammengefasst sind.

Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind solche Vitamine, Provitamine und Vitaminvorstufen bevorzugt, die üblicherweise den Gruppen A, B, C, E, F und H zugeordnet werden. Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die Mittel weiterhin mindestens einen Pflanzenextrakt enthalten.

Auch Mono- bzw. Oligosaccharide können als Pflegestoff in den Mitteln eingesetzt werden. Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten. Die Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.
Zu den natürlichen und synthetischen kosmetischen Ölkörpern sind beispielsweise zu zählen:
- pflanzliche Öle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen.
- Esteröle. Unter Esterölen sind zu verstehen die Ester von C₆ - C₃₀ - Fettsäuren mit C₂ - C₃₀ - Fettalkoholen.
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen.
- Trifettsäureester von gesättigten und/oder ungesättigten linearen und/oder verzweigten Fettsäuren mit Glycerin,
   Fettsäurepartialglyceride, worunter Monoglyceride, Diglyceride und deren technische Gemische zu verstehen sind.

Die Einsatzmenge der natürlichen und synthetischen kosmetischen Ölkörper in den Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt. Die Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Vorzugsweise werden die Mittel als Pumpspray, Aerosolspray, Pumpschaum oder Aerosolschaum. Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird. Insbesondere bevorzugt sind die Mittel als Aerosolhaarschaum oder Aerosolhaarspray konfektioniert. Das erfindungsgemäße Mittel enthält daher bevorzugt zusätzlich mindestens ein Treibmittel. Der Gegenstand der Erfindung ist die Verwendung der erfindungsgemäßen Mittel des zweiten Erfindungsgegenstandes zur temporären Verformung von Haaren und gleichzeitiger Erzeugung von Glanz.

Dabei ist es erfindungsgemäß bevorzugt, dass die keratinhaltigen Fasern in Form gebracht werden und diese Form durch das besagte Mittel, fixiert wird.

Als erfindungsgemäß bevorzugt gelten für diesen Gegenstand ebenso die zuvor genannten Ausführungsformen der Verwendung der Cycloalkane.

### Beispiele

Das folgende Stylingmittel wurde durch Mischen der Komponenten bereitgestellt:

**Tabelle 1: Stylingemulsion**

| Rohstoff | E1 | E2 |
|---|---|---|
| | [Gew.-%] | [Gew.-%] |
| Oleth-10 (Oleylalkohol mit 10 Einheiten Ethylenoxid verethert) | 7,50 | 7,50 |
| Oleth-20 (Oleylalkohol mit 20 Einheiten Ethylenoxid verethert) | 5,00 | 5,00 |
| Isopropylmyristat | 5,00 | 5,00 |
| Polyquaternium-4 | 0,05 | - |
| 1,4-Dihydroxymethylcyclohexan | 3,60 | 3,60 |
| Hydriertes Rizinusöl mit 25 Einheiten Ethylenoxid | 15,00 | 15,00 |
| Glycerin | 5,00 | 5,00 |
| 1,2-Propandiol | 20,00 | 20,00 |
| Parfum | 0,50 | 0,50 |
| Wasser, vollentsalzt | ad 100 | ad 100 |

Je ein Mittel der Tabelle 1 wurden auf jeweils eine Haarsträhne appliziert und auf der Haarsträhne belassen. Die Form der Haarsträhnen wurde fixiert und das Haar erhielt einen natürlichen Glanz.

## Patentansprüche

1. Verwendung eines Mittels zur temporären Umformung keratinhaltiger Fasern, insbesondere menschlicher Haare, wobei das Mittel in einem kosmetisch akzeptablen Träger
a) 1,4-Bis(hydroxymethyl)cyclohexan
b) mindestens einen festigenden Wirkstoff
enthält.

2. Verwendung nach Anspruch 1, **dadurch gekennzeichnet, dass** das 1,4-Bis(hydroxymethyl)-cyclohexan in einer Menge von 0,5 bis 20,0 Gew.-%, insbesondere von 1,0 bis 10,0 Gew.-%, jeweils bezogen auf das Gewicht des Mittels, enthalten ist.

3. Verwendung nach einem der Ansprüche 1 bis 2, **dadurch gekennzeichnet, dass** der festigende Wirkstoff ausgewählt wird aus mindestens einem festigenden Polymer und/oder mindestens einem Wachs.

4. Verwendung nach einem der Ansprüche 1 bis 3, **dadurch gekennzeichnet, dass** der festigende Wirkstoff in einer Menge von 0,1 Gew.-% bis 25,0 Gew.-%, besonders bevorzugt von 1,0 Gew.-% bis 20,0 Gew.-%, ganz besonders bevorzugt von 1,0 Gew.-% bis 10,0 Gew.-%, jeweils bezogen auf das Gewicht des Mittels, enthalten ist.

## Claims

1. The use of an agent for temporarily shaping keratin-containing fibers, in particular human hair, wherein the agent contains, in a cosmetically acceptable carrier,
a) 1,4-bis(hydroxymethyl)cyclohexane
b) at least one stabilizing active substance.

2. The use according to claim 1, **characterized in that** the 1-4-bis(hydroxymethyl)-cyclohexane is contained in an amount of from 0.5 to 20.0 wt.%, in particular 1.0 to 10.0 wt.%, in each case based on the weight of the agent.

3. The use according to one of claims 1 to 2, **characterized in that** the stabilizing active ingredient is selected from at least one stabilizing polymer and/or at least one wax.

4. The use according to one of claims 1 to 3, **characterized in that** the stabilizing active ingredient is contained in an amount of from 0.1 wt.% to 25.0 wt.%, particularly preferably 1.0 wt.% to 20.0 wt.%, most preferably 1.0 wt.% to 10.0 wt.%, in each case based on the weight of the agent.

## Revendications

1. Utilisation d'un produit de déformation temporaire de fibres de kératine, en particulier de cheveux humains, le produit contenant dans un vecteur cosmétiquement acceptable :
a) du 1,4-bi(hydroxyméthyl)cyclohexane
b) au moins un agent actif solidifiant.

2. Utilisation selon la revendication 1, **caractérisée en ce que** le 1,4-bi(hydroxyméthyl)cyclohexane est contenu à hauteur de 0,5 à 20,0 % en poids, en particulier de 1,0 à 10,0 % en poids, respectivement rapporté au poids du produit.

3. Utilisation selon la revendication 1 à 2, **caractérisée en ce que** l'agent actif est choisi parmi au moins un polymère solidifiant et/ou au moins une cire.

4. Utilisation selon la revendication 1 à 3, **caractérisée en ce que** l'agent actif est contenu à hauteur de 0,1 à 25,0 % en poids, particulièrement préférentiellement de 1,0 à 20,0 % en poids, tout particulièrement préférentiellement de 1,0 à 10,0 % en poids, respectivement rapporté au poids du produit.
